# EUROPÄISCHE PATENTSCHRIFT

(11) **EP 0 564 944 B1**
(45) Veröffentlichungstag und Bekanntmachung des Hinweises auf die Patenterteilung: **08.05.1996**
(21) Anmeldenummer: 93105147.8
(22) Anmeldetag: 29.03.1993
(51) Int. Cl.: C07C 17/00, C07C 21/18

(54) **Verfahren zur Herstellung von Chlor-Fluor-Butenen**
Process for the preparation of chloro-fluoro-butenes
Procédé de préparation de chloro-fluoro-butènes

(30) Priorität: 10.04.1992 DE 4212084
(43) Veröffentlichungstag der Anmeldung: 13.10.1993
(73) Patentinhaber: BAYER AG, D-51368 Leverkusen (DE)
(72) Erfinder: Lui, Norbert, Dr., W-5000 Köln 80 (DE); Marhold, Albrecht, Dr., W-5090 Leverkusen 1 (DE); Bielefeldt, Dietmar, Dr., W-4030 Ratingen 6 (DE)

(56) Entgegenhaltungen:
- EP-A- 0 301 346
- DE-B- 1 278 429
- US-A- 3 965 201

## Beschreibung

Die vorliegende Erfindung betrifft ein Verfahren zur gleichzeitigen Herstellung von 2-Chlor- und 2,3-Dichlor-1,1,1,4,4,4-hexafluorbuten-2 aus Chlorbutenderivaten.

2-Chlor-1,1,1,4,4,4-hexafluorbuten-2 ist ein bekanntes Zwischenprodukt zur Herstellung von Natriumtrifluoroacetat. Als Herstellungsmethoden sind die Umsetzung von Hexachlorbutadien mit Fluorwasserstoff und Chlor unter Zugabe von Antimonpentachlorid (US-PS 2 436 357 und C.A. 46, 7987 i bis 7988 a) und die Umsetzung von Hexachlorbutadien mit Fluorwasserstoff unter Zugabe katalytischer Mengen von Titantetrahalogenid, Antimontrihalogenid und/oder Antimonpentachlorid (DE-OS 37 25 213) bekannt.

2,3-Dichlor-1,1,1,4,4,4-hexafluorbuten-2 ist ein bekanntes Zwischenprodukt zur Herstellung von Hexafluorbutan, einem Wärmeübertragungsmittel, sowie von Trifluoressigsäure. Es kann aus Hexachlorbutadien durch Umsetzung mit Fluorwasserstoff und Chlor unter Zugabe von Antimonpentachlorid hergestellt werden (US-PS 2 436 357 und C.A. 46, 7987 i bis 7988 a).

Das für diese Verfahren als Ausgangsmaterial benötigte Hexachlorbutadien wird neuerdings nicht mehr in technischem Maßstab hergestellt, da es in den Verdacht geraten ist, krebserregend zu sein. Es besteht deshalb ein Bedürfnis nach einem Verfahren zur Herstellung von 2-Chlor- und 2,3-Dichlor-1,1,1,4,4,4-hexafluorbuten-2, bei dem in technischem Maßstab herstellbare Ausgangsprodukte verwendet werden.

Aus der US-PS 3 965 201 ist bekannt, daß man aus Hexachlorbutadien durch Umsetzung mit Fluorwasserstoff und Chlor 2,3-Dichlorhexafluorbuten-2 erhält.

Es wurde nun ein Verfahren zur gleichzeitigen Herstellung von 2-Chlor- und 2,3-Dichlor-1,1,1,4,4,4-hexafluorbuten-2 gefunden, das dadurch gekennzeichnet ist, daß man ein Chlorbutenderivat der Formel (I)

Cl₂C=CH-X (I),

in der
- X: für CCl=CCl₂ oder CCl₂-CCl₂H steht,
mit Fluorwasserstoff und Chlor in Gegenwart eines Katalysators bei Temperaturen im Bereich 50 bis 550°C umsetzt.

Die für dieses Verfahren benötigten Ausgangsverbindungen der Formel (I) sind z.B. zugänglich gemäß Zh. Org. Khim. 17(2), 272 bis 275.

Die anderen benötigten Ausgangsprodukte, Fluorwasserstoff und Chlor, werden vorzugsweise in wasserfreier Form eingesetzt und sind als solche im Handel erhältlich.

Der Fluorwasserstoff kann beispielsweise in Mengen von 5 bis 100 Mol, bezogen auf 1 Mol eines Chlorbutenderivats der Formel (I) verwendet werden. Bevorzugt beträgt diese Menge 10 bis 50 Mol pro Mol Chlorbutenderivat der Formel (I).

Das Chlor kann beispielsweise in Mengen von 1 bis 3 Mol bezogen auf 1 Mol Chlorbutenderivat der Formel (I) verwendet werden. Bevorzugt beträgt diese Menge 1,1 bis 2,2 Mol pro Mol Chlorbutadienderivat der Formel (I).

Man kann das erfindungsgemäße Verfahren in der flüssigen und in der Gasphase durchführen Beim Arbeiten in der flüssigen Phase, d.h. bei entsprechend tiefen Temperaturen und/oder bei erhöhtem Druck, kommen für Chlor/Fluor-Austauschreaktionen in der Flüssigphase übliche Katalysatoren in Frage, beispielsweise Titanhalogenide, insbesondere Titantetrachlorid, Niobhalogenide, insbesondere Niobpentachlorid, Tantalhalogenide, insbesondere Tantalpentachlorid, Antimontrifluorid, Antimonpentafluorid, Antimonpentachlorid und/oder gemischte Antimonpentahalogenide, beispielsweise solche der summarischen Formel SbClₙF₅₋ₙ mit n = 0,1 bis 4,9. Solche Katalysatoren können jeweils alleine oder in beliebigen Gemischen untereinander eingesetzt werden. Gegebenenfalls kann Fluorsulfonsäure als Cokatalysator eingesetzt werden.

Wenn das erfindungsgemäße Verfahren in der Gasphase durchgeführt wird, d.h. bei entsprechend hohen Temperaturen und niedrigen Drucken, können für Chlor/Fluor-Austauschreaktionen in der Gasphase übliche Katalysatoren eingesetzt werden. Beispielsweise kommen in Frage Halogenide und Oxide von Metallen und Übergangsmetallen. Geeignet sind insbesondere Chloride, Fluoride und/oder gegebenenfalls gemischte, Oxide von Kupfer, Chrom, Eisen, Wismut, Zink, Lanthan, Cer, Zirkon, Vanadin, Molybdän, Wolfram und/oder Nickel. Bevorzugt sind Chrom(III)-salze alleine oder im Gemisch mit Chloriden der anderen genannten Metalle und/oder deren Fluoriden und/oder deren Oxiden. Im Gasphasenverfahren können die Katalysatoren als solche, z.B. in stückiger Form, verwendet werden, aber auch aufgebracht auf einen Träger, beispielsweise auf Aluminiumoxid, Magnesiumoxid, Magnesiumfluorid, Calciumfluorid, Zinkchlorid und/oder Aktivkohle.

Die Menge, in der beim erfindungsgemäßen Verfahren Katalysatoren eingesetzt werden, ist nicht kritisch. Aus wirtschaftlichen Erwägungen wählt man die Katalysatormenge vorteilhafterweise so, daß ein Umsatz von mindestens 60 % erreicht wird. Beim Arbeiten in der Gasphase kann man beispielsweise pro Stunde 50 g bis 5 kg des Chlor-butenderivates der Formel (I) über einen Liter des Katalysators leiten. Beim Arbeiten in der Flüssigphase kann man beispielsweise 0,1 bis 30 Gew.-% Katalysator, bezogen auf das eingesetzte Chlor-butenderivat der Formel (I), verwenden.

Wenn man in flüssiger Phase arbeiten möchte, so muß in jedem Fall in geschlossenen Gefäßen oder unter Druck gearbeitet werden, da Fluorwasserstoff bei Normaldruck bei ungefähr 20°C siedet. Für das Arbeiten in der Flüssigphase geeignete Reaktionsbedingungen sind beispielsweise Temperaturen von 50 bis 200°C und Drucke von 6 bis 50 bar.

Bei Durchführung des erfindungsgemäßen Verfahrens in der Gasphase kann die Temperatur beispielsweise im Bereich von 200 bis 550°C liegen. Für die Gasphase bevorzugte Reaktionsbedingungen sind Temperaturen von 250 bis 480°C und Drucke im Bereich 0,5 bis 3 bar.

Bei diskontinuierlicher Durchführung des erfindungsgemäßen Verfahrens, insbesondere in der Flüssigphase, ist es im allgemeinen vorteilhaft, nach Beendigung der Reaktion noch einige Zeit bei der zuletzt angewendeten Temperatur nachzurühren, beispielsweise 1 bis 5 Stunden. Bei kontinuierlicher Durchführung des erfindungsgemäßen Verfahrens, insbesondere in der Gasphase, ist es im allgemeinen vorteilhaft, den Gasstrom nach dem Passieren des Katalysators für kurze Zeit, beispielsweise 5 bis 30 Minuten auf Reaktionstemperatur zu halten.

Das nach Beendigung der Reaktion vorliegende Reaktionsgemisch, kann, gegebenenfalls nach Abkühlung und Kondensation, beispielsweise so aufgearbeitet werden, daß man zunächst noch vorhandenen Fluorwasserstoff abtrennt, z.B. durch Phasentrennung oder durch Destillation, und den Rückstand fraktioniert destilliert oder den vom Fluorwasserstoff befreiten Rückstand auf Eis gibt, die sich bildende organische Phase abtrennt und diese fraktioniert destilliert. Soweit im Reaktionsgemisch lösliche Katalysatoren eingesetzt worden sind, kann man diese gegebenenfalls zurückgewinnen, beispielsweise indem man das Reaktionsgemisch nach der Entfernung des Fluorwasserstoffs mit einer Weinsäurelösung extrahiert.

Gegenüber den bekannten Verfahren zur Herstellung von 2-Chlor- und 2,3-Dichlor-1,1,1,4,4,4-hexafluorbuten-2 hat man den Vorteil, daß es nicht von dem nicht mehr technisch zugänglichen Hexachlorbutadien ausgeht, sondern von Chlorbutenderivaten der Formel (I), die sich leicht und in großen Mengen aus Trichlorethylen herstellen lassen.

Es konnte nicht vorhergesehen werden, daß die Umsetzung von Chlorbutenderivaten der Formel (I) mit Fluorwasserstoff und Chlor sowohl in der Flüssigphase, als auch in der Gasphase so gute Ergebnisse erbringt, da, im Gegensatz zu den Verhältnissen beim Einsatz von Hexachlorobutadien mit Nebenreaktionen und Spaltungen zu rechnen war.

### Beispiel 1

110,4 g BiCl₃ wurden in 150 g 18 gew.-%iger wäßriger Salzsäure gelöst und diese Lösung dann mit einer Lösung von 16,76 g FeCl₃ x 6 H₂O in 30 g Wasser vermischt. In einem Kneter wurden 250 g MgO vorgelegt und die Lösung der Metallsalze hinzugefügt. Während des Knetens wurden noch 170 ml Wasser zugesetzt. Nach einer Knetzeit von 1,5 Stunden wurde die geknetete Masse getrocknet, gemahlen, mit 2 Gew.-% Graphit versetzt und gepillt. Das Atomverhältnis Mg : Bi : Fe betrug 1:0,06:0,04.

In einem Nickelrohr, das von außen elektrisch beheizbar war und eine lichte Weite von 30 mm aufwies, wurden 350 ml des so hergestellten Katalysators eingefüllt und bei 350°C im Verlaufe von 3 Stunden 5 Mol Fluorwasserstoff hindurchgeleitet. Zum Einsatz kam ein Gemisch von Fluorwasserstoff mit Stickstoff im Molverhältnis 1:2.

Anschließend wurden bei 460°C 80 g Hexachlorbuten (Formel (I), X = CCl₂-CCl₂H), 28 g Chlor und 400 ml Fluorwasserstoff (gemessen als Flüssigkeit) pro Stunde über den Katalysator geleitet. Die aus dem Reaktionsrohr austretenden Gase wurden abgekühlt und in einer auf -50°C gekühlten Vorlage kondensiert. Nach 2 Stunden Betrieb wurde die Zufuhr von Hexachlorbuten, Chlor und Fluorwasserstoff abgestellt und nach weiteren 30 Minuten der Inhalt der Vorlage einer Phasentrennung unterworfen. Die untere, organische Phase wurde auf 200 g Eis geschüttet und nach dem Erwärmen auf 10°C erneut von der wäßrigen Phase getrennt. Es wurden 112 g Reaktionsprodukt produkt erhalten, das 18,5 Gew.-% 2-Chlor-1,1,1,4,4,4-hexafluor-2-buten (cis/trans) und 57,5 Gew.-% 2,3-Dichlor-1,1,1,4,4,4-hexafluor-2-buten enthielt.

### Beispiel 2

1145 ml Wasser und 588 g 28 gew.-%ige wäßrige Ammoniaklösung wurden in einen Kolben eingegeben, der mit einem mechanischen Rührer und einem Tropftrichter versehen war. Durch den Tropftrichter wurden im Verlauf von einer Stunde 520 g einer 37 gew.-%igen wäßrigen Lösung von Chrom(III)-chlorid und 1 Liter Wasser zugegeben. Der sich ergebende Niederschlag wurde abgetrennt, mit Wasser gewaschen und bei 70°C in einen Vakuumofen eingestellt. Die sich ergebende, noch feuchte, wasserhaltiges Chrom(III)-oxid enthaltende Paste wurde in kleine Würfel zerschnitten und abschließend bei 480°C in einer Stickstoffatmosphäre getrocknet.

350 ml dieses Katalysators wurden wie in Beispiel 1 beschrieben mit Fluorwasserstoff vorbehandelt und anschließend wie in Beispiel 1 beschrieben mit 80 g Hexachlorbuten, 30 g Chlor und 220 g Fluorwasserstoff beaufschlagt. Es wurden 106 g eines Reaktionsprodukts erhalten, das 12,7 Gew.-% 2-Chlor-1,1,1,4,4,4-hexafluor-2-buten und 61,3 Gew.-% 2,3-Dichlor-1,1,1,4,4,4-hexafluor-2-buten enthielt.

### Beispiel 3

CrCl₃ x 6 H₂O wurde in Wasser aufgelöst. Diese Lösung wurde mit Magnesiumoxid und Graphit versetzt und die entstehende pastöse Masse verknetet. Das erhaltene pastöse Reaktionsprodukt wurde zu Würfeln mit einer Kantenlänge von 0,5 cm zerkleinert und dann 16 Stunden bei 100°C getrocknet. Der fertige Katalysator enthielt 17 Gew.-% CrCl₃, 76 Gew.-% MgO und 7 Gew.-% Graphit.

Dieser Katalysator wurde wie in Beispiel 1 beschrieben mit Fluorwasserstoff behandelt und anschließend wie in Beispiel 1 beschrieben mit 80 g Hexachlorbuten, 28 g Chlor und 400 ml Fluorwasserstoff pro Stunde beaufschlagt.

Es wurden 117,5 g eines Reaktionsprodukts erhalten, das 11,6 Gew.-% 2-Chlor-1,1,1,4,4,4-hexafluor-2-buten und 66,1 Gew.-% 2,3-Dichlor-1,1,1,4,4,4-hexafluor-2-buten enthielt.

### Beispiel 4

300 g CrCl₃ x 6 H₂O und 30 g MgF₂ wurden in 10 l Wasser eingetragen und auf 90°C erwärmt. Nach 1 Stunde wurden 1300 g einer 11 gew.-%igen wäßrigen Ammoniaklösung zudosiert. Anschließend wurde 1 Stunde nachgerührt, abkühlen gelassen und der ausgefallene Feststoff über eine Filternutsche abfiltriert. Der Feststoff wurde zwei mal mit Wasser gewaschen, getrocknet, pulverisiert und mit 2 Gew.-% Graphit homogen vermischt. Dieses Gemisch wurde zu 4 mm großen Tabletten verpreßt.

Der so erhaltene Katalysator wurde wie in Beispiel 1 beschrieben mit Fluorwasserstoff vorbehandelt und anschließend wie in Beispiel 1 beschrieben mit 80 g Pentachlorbutadien, 27 g Chlor und 230 g Fluorwasserstoff pro Stunde beaufschlagt. Es wurden 98 g eines Reaktionsprodukts erhalten, das 19,2 Gew.-% 2-Chlor-1,1,1,4,4,4-hexafluor-2-buten und 53,4 Gew.-% 2,3-Dichlor-1,1,1,4,4,4-hexafluor-2-buten enthielt.

### Beispiel 5

400 g Hexachlorbuten wurden unter Zusatz von 2 ml Antimonpentachlorid mit 1,7 Mol Chlor bei 40°C chloriert. Anschließend wurde das Gemisch in einen Autoklaven gebracht. Dort wurden 600 ml wasserfreier Fluorwasserstoff und weitere 50 g Antimonpentachlorid zugefügt. Die Reaktionsmischung wurde bei dem sich von selbst einstellenden Druck 5 Stunden lang auf 140°C erhitzt, wobei entstehender Chlorwasserstoff so entspannt wurde, daß der Fluorwasserstoff in flüssiger Phase verblieb. Nach dem Abkühlen auf Raumtemperatur wurde das Gemisch auf Eis gegossen und die organische Phase abgetrennt. Es wurden 240 g Reaktionsprodukt erhalten, das 20 Gew.-% 2-Chlor-1,1,1,4,4,4-hexafluorbuten enthielt, Die minderfluorierten Anteile können wieder als Edukt in die Fluorierung eingesetzt werden.

## Patentansprüche

1. Verfahren zur gleichzeitigen Herstellung von 2-Chlor- und 2,3-Dichlor-1,1,1,4,4,4-hexafluorbuten-2, dadurch gekennzeichnet, daß man ein Chlorbutenderivat der Formel (I)
Cl₂C=CH-X (I),
in der
X für CCl=CCl₂ oder CCl₂-CCl₂H steht,
mit Fluorwasserstoff und Chlor in Gegenwart eines Katalysators bei Temperaturen im Bereich 50 bis 550°C umsetzt`

2. Verfahren nach Anspruch 1, dadurch gekennzeichnet, daß man es in der Gasphase bei 200 bis 550°C und in Gegenwart eines Katalysators durchführt, der Halogenide und/oder Oxide von Metallen und/oder Übergangsmetallen enthält.

3. Verfahren nach Anspruch 1, dadurch gekennzeichnet, daß man es in der flüssigen Phase bei Temperaturen von 50 bis 200°C, Drucken von 6 bis 50 bar und in Gegenwart von Titanhalogeniden, Niobhalogeniden, Tantalhalogeniden, Antimontrifluorid, Antimonpentafluorid, Antimonpentachlorid und/oder gemischten Antimonpentahalogeniden als Katalysator durchführt.

4. Verfahren nach Ansprüchen 1 bis 3, dadurch gekennzeichnet, daß man 5 bis 100 Mol Fluorwasserstoff und 1 bis 3 Mol Chlor jeweils bezogen auf 1 Mol Chlorbutenderivat der Formel (I) einsetzt.

5. Verfahren nach Anspruch 2, dadurch gekennzeichnet, daß man beim Arbeiten in der Gasphase Chrom(III)salze enthaltende Katalysatoren einsetzt.

## Claims

1. Process for the simultaneous preparation of 2-chloro- and 2,3-dichloro-1,1,1,4,4,4-hexafluoro-2-butene, characterised in that a chlorobutene derivative of the formula (I)
Cl₂C=CH-X (I)
in which
X represents CCl=CCl₂ or CCl₂-CCl₂H
is reacted with hydrogen fluoride and chlorine in the presence of a catalyst at temperatures in the range of 50 to 550°C.

2. Process according to Claim 1, characterised in that it is carried out in the gas phase at from 200 to 550°C and in the presence of a catalyst which comprises halides and/or oxides of metals and/or transition metals.

3. Process according to Claim 1, characterised in that it is carried out in the liquid phase at temperatures from 50 to 200°C, pressures from 6 to 50 bar, and in the presence of titanium halides, niobium halides, tantalum halides, antimony trifluoride, antimony pentafluoride, antimony pentachloride and/or mixed antimony pentahalides as a catalyst.

4. Process according to Claims 1 to 3, characterised in that from 5 to 100 mol of hydrogen fluoride and from 1 to 3 mol of chlorine are used, each based on 1 mol of chlorobutene derivative of the formula (I).

5. Process according to Claim 2, characterised in that the reaction in the gas phase employs catalysts containing chromium (III) salts.

## Revendications

1. Procédé pour la préparation simultanée de 2-chloro- et 2,3-dichloro-1,1,1,4,4,4-hexafluorobutène-2, caractérisé en ce qu'on fait réagir un dérivé de chlorobutène de formule (I)
Cl₂C=CH-X (I)
dans laquelle
X représente CCl=CCl₂ ou CCl₂-CCl₂H
avec de l'acide fluorhydrique et du chlore en présence d'un catalyseur, à des températures dans le domaine de 50 à 550°C.

2. Procédé selon la revendication 1, caractérisé en ce qu'on l'effectue en phase gazeuse à une température de 200 à 550°C et en présence d'un catalyseur qui contient des halogénures et/ou des oxydes de métaux et/ou de métaux de transition.

3. Procédé selon la revendication 1, caractérisé en ce qu'on l'effectue en phase liquide à des températures de 50 à 200°C, sous des pressions de 6 à 50 bar et en présence d'halogénures de titane, d'halogénures de niobium, d'halogénures de tantale, de trifluorure d'antimoine, de pentafluorure d'antimoine, de pentachlorure d'antimoine et/ou de pentahalogénures mixtes d'antimoine, comme catalyseur.

4. Procédé selon les revendications 1 à 3, caractérisé en ce qu'on met en oeuvre de 5 à 100 moles d'acide fluorhydrique et de 1 à 3 moles de chlore, respectivement rapportées à 1 mole du dérivé de chlorobutène de formule (I).

5. Procédé selon la revendication 2, caractérisé en ce qu'en travaillant en phase gazeuse, on met en oeuvre des catalyseurs contenant des sels de chrome(III).
